(19) Europäisches Patentamt
European Patent Office
Office européen des brevets

(11) Publication number: **0 199 963 B1**

(12) # EUROPEAN PATENT SPECIFICATION

(45) Date of publication of patent specification: **23.10.91**

(51) Int. Cl.5: **G01N 33/58**, G01N 33/533, C07D 493/10, C07K 15/00

(21) Application number: **86103673.9**

(22) Date of filing: **18.03.86**

(54) **Ethosuximide assay tracers, immunogens and antibodies.**

(30) Priority: **01.04.85 US 718601**

(43) Date of publication of application:
**10.12.86 Bulletin 86/45**

(45) Publication of the grant of the patent:
**23.10.91 Bulletin 91/43**

(84) Designated Contracting States:
**BE DE FR IT**

(56) References cited:
**FR-A- 2 276 317**
**FR-A- 2 500 165**
**FR-A- 2 518 096**
**GB-A- 2 081 257**

(73) Proprietor: **ABBOTT LABORATORIES**
**14th Street and Sheridan Road North St**
**North Chicago, Illinois 60064(US)**

(72) Inventor: **Heiman, Daniel Feulner**
**407 Drake**
**Libertyville Illinois 60048(US)**
Inventor: **Cantarero, Luis A.**
**1319 Dunleer**
**Mundelein Illinois 60060(US)**
Inventor: **Chan, Clifford Man**
**17652 West Windslow Drive**
**Grayslake Illinois 60030(US)**

(74) Representative: **Modiano, Guido et al**
**MODIANO, JOSIF, PISANTY & STAUB**
**Modiano & Associati Via Meravigli, 16**
**I-20123 Milano(IT)**

**EP 0 199 963 B1**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).

## Description

1. Technical Field

The present invention relates to a method and reagents for a fluorescence polarization immunoassay procedure for determining the amount of ethosuximide in fluids, especially biological fluids such as serum, plasma or urine, and to a method of making the reagents. More specifically, the invention relates to (1) reagents (tracers and antibodies) for determining the amount of ethosuximide in a sample; (2) immunogen compounds used to raise antibodies; (3) synthetic methods (for making the tracer and immunogen compounds); and (4) analytical methods for conducting the assay.

2. Background Art

Ethosuximide cf. Figure 1 of the drawings, is a drug used for the treatment of seizures caused by petit mal epilepsy. The frequency of convulsions is apparently reduced by depression of the motor cortex and elevation of the threshold of the central nervous system to convulsive stimuli. Ethosuximide can produce serious adverse reactions of the gastrointestinal system, the hemopoietic system and the nervous system, among other side effects. Plasma levels between 40 and 100 micrograms per milliliter (µg/ml) are believed to be therapeutically effective and levels greater than 100 µg/ml are believed to be toxic. The plasma level of ethosuximide varies widely among patients due to wide variation in individual pharmacokinetics. In addition, the kinetics can change in the same patient as the condition of the patient changes. Therefore, monitoring the ethosuximide level in patient blood is recommended for safe and effective therapy.

In the past, patient serum or plasma ethosuximide levels have typically been measured by high performance liquid chromatography (HPLC), gas chromatography (GC), enzyme immunoassay (EIA) and substrate-linked fluorescence immunoassay (SLFIA). These methods are reasonably specific for detecting drug levels; however, they are not without drawbacks. HPLC and GC methods require sample extraction procedures and the assay time is lengthy. Both EIA and SLFIA involve enzyme reactions and have the following disadvantages: 1) the reagents are relatively unstable; 2) any components in the biological samples which may influence the enzyme reaction in EIA or SLFIA (such as enzyme inhibitors or enzymes which catalyze similar reactions) will affect the assay results; and 3) EIA and SLFIA measure either absorbance or fluorescence, and any compounds in the biological samples which may affect absorbance or fluorescence (such as lipid, hemoglobin, bilirubin or other chromophores or fluorophores) will affect the accuracy of the results obtained from these assays.

In assays for other substances, competitive binding immunoassays have provided a more satisfactory alternative. Typically, competitive binding immunoassays are used for measuring ligands in a test sample. (For purposes of this disclosure, a "ligand" is a substance of biological interest to be determined quantitatively by a competitive binding immunoassay technique). The ligands compete with a labeled reagent, or "ligand analog," or "tracer," for a limited number of receptor binding sites on antibodies specific to the ligand and ligand analog. The concentration of ligand in the sample determines the amount of ligand analog which binds to the antibody: the amount of ligand analog that will bind is inversely proportional to the concentration of ligand in the sample, because the ligand and the ligand analog each bind to the antibody in proportion to their respective concentrations.

Fluorescence polarization provides a quantative means for measuring the amount of tracer-antibody conjugate produced in a competitive binding immunoassay. Fluorescence polarization techniques are based on the principle that a fluorescent labeled compound, when excited by plane polarized light, will emit fluorescence having a degree of polarization inversely related to its rate of rotation. Accordingly, when a tracer-antibody conjugate having a fluorescent label is excited with plane polarized light, the emitted light remains highly polarized because the fluorophore is constrained from rotating between the time that light is absorbed and emitted. In contrast, when an unbound tracer is excited by plane polarized light, its rotation is much faster than that of the corresponding tracer-antibody conjugate. As a result, the light emitted from the unbound tracer molecules is depolarized.

Such fluorescence polarization techniques have been applied in U.S. Patent No. 4,420,568 to Wang, et al., which is directed to the use of a triazinylamino-fluorescein moiety as the fluorophore. Ethosuximide antigen conjugates and antibodies are described in Canadian Patent No. 1,037,475 to Soffer, et al. and in EP-A-0 095 665 by Buckler, et al. A fluorescence polarization immunoassay of ethosuximide is described in a 1983 technical publication entitled, "Polarization Fluoroimmunoassay of Ethosuximide", available from International Laboratory Services, London, England.

The present invention offers an advance in the art beyond the foregoing art, in that highly sensitive

tracers, a method for making the tracers, and an assay using the tracers are provided specifically for the determination of ethosuximide. The assay conducted in accordance with the present invention is particularly accurate, as will be explained infra.

## SUMMARY OF THE INVENTION

The present invention is directed to a fluorescence polarization assay for ethosuximide; to tracers, immunogens and antibodies for use in the assay; and to methods for making the tracers, immunogens and antibodies.

A first aspect of the invention relates to the finding of unique tracers and immunogens having novel structures. According to the first aspect of the invention, the tracers and the immunogens can both be represented by the structural formula shown in Figure 2 of the drawings wherein:

$R_1$ is -H or -R-Z-Q;

$R_2$ is $-CH_2$-R-Z-Q when $R_1$ is H and $R_2$ is methyl or ethyl when $R_1$ is -R-Z-Q;

$R_3$ is methyl or ethyl;

Q is a poly(aminoacid), or another immunologically active carrier selected from natural proteins, synthetic poly(amino acids) having a sufficient number of available carboxylate groups, carbohydrates, yeasts and polysaccharides or fluorescein or a fluorescein derivative;

Z is NH, CO, CS, $SO_2$ C=NH, O or a single bond when Q is said fluorescein or a fluorescein derivative and NH-NH, NH, N=N or $CH_2$ when Q is said poly(aminoacid), or said other immunologically active carrier; and

R is a group consisting of from 0 to 8 carbon atoms arranged in a straight or branched chain or in a phenylene or alkylenephenylene moiety, and with the proviso than when $R_1$ is R-Z-Q, Q is an amino derivative of fluorescein, Z is CO and R is a normal alkylene chain, then R must contain at least three carbon atoms.

When Q is a poly(amino acid),

or any other immunologically active carrier the compound can be used as an immunogen. When Q is fluorescein or a derivative thereof, the compound can be used as a tracer.

A second aspect of the invention relates to antibodies raised by the novel immunogen. According to the second aspect of the invention, antibodies are prepared in response to a compound according to claim 1 when Q is a poly(amino acid) or other immunologically active carrier.

According to a third aspect of the invention, an immunogen is made by a method comprising the step of coupling a compound represented by the structural formula shown in Figure 2 wherein:

$R_1$ is -H or -R-X;

$R_2$ is $-CH_2$-R-X when $R_1$ is H and $R_2$ is methyl or ethyl when $R_1$ is -R-X;

$R_3$ is methyl or ethyl;

X is $NH_2$, Cl, Br, I , OH or CHO; and

R is a group consisting of a total of from 0 to 8 carbon atoms arranged in a straight or branched chain or in a phenylene or alkylenephenylene moiety

with a poly(amino acid), or another immunologically active carrier selected from natural proteins, synthetic poly(amino acids) having a sufficient number of available carboxylate groups, carbohydrates, yeasts and polysaccharides.

According to a fourth aspect of the invention, a method is provided for making a tracer by coupling a compound represented by the structural formula shown in Figure 2, wherein:

$R_1$ is -H or -R-Y;

$R_2$ is $-CH_2$-R-Y when $R_1$ is H and $R_2$ is methyl or ethyl when $R_1$ is -R-Y;

$R_3$ is methyl or ethyl;

Y is $-NH_2$, -COOH, $-SO_3H$, $-SO_2Cl$, -SH, -CHO, -CN or -OH;

R is a group consisting of from 0 to 8 carbon atoms arranged in a straight or branched chain or in a phenylene or alkylenephenylene moiety, with the proviso that when $R_1$ is -R-Y, R is a normal alkylene chain and Y is COOH, then R must contain at least three carbon atoms

with fluorescein or a derivative of fluorescein.

According to a fifth aspect of the invention, a process for measuring concentration of ethosuximide is provided. A sample is contacted, in the presence of a buffer to achieve and maintain the pH, with ethosuximide antiserum and with an ethosuximide derivative containing fluorescein in the open tautomeric form capable of producing a detectable fluorescence polarization response to the presence of the ethosuximide antiserum. Plane polarized light is then passed through the solution to obtain a fluorescence polarization response, and this response is detected as a measure of the amount of ethosuximide in the

sample.

Further objects and attendant advantages of the invention will be best understood from a reading of the following detailed description taken together with the Figures and the Examples.

## BRIEF DESCRIPTION OF THE DRAWINGS

In the following figures the symbol "Fl" represents a fluorescein moiety, and the various other symbols are noted in the Detailed Description. Note that the orientation of $R_2$ and $R_3$ and/or the methyl and ethyl groups, in the Figures is not meant to imply nor to exclude any particular enantiomer or diastereomer.

Figure 1 shows the structure of the drug ethosuximide to be quantitatively determined in accordance with the present invention.

Figure 2 shows a general structural formula for the tracers and the immunogens of the present invention as well as the classes of reactants used in preparing them.

Figure 3 shows the alternate structural formulae and names of the fluorescein moiety included in the tracers of the present invention.

Figure 4 shows various linkages that couple the fluorescein moiety to the precursor in Figure 2, when Figure 2 represents a precursor for the tracers.

Figures 5 to 20 show various examples of structures of tracers in accordance with the present invention.

Figures 21 to 24 show various examples of structures of hapten reactants used to form the immunogens employed in the present invention.

## DETAILED DESCRIPTION OF THE INVENTION

The various aspects of the invention will now be discussed in detail in relation to the drawing figures.

The present invention involves the use of fluorescein and derivatives of fluorescein. In particular, a necessary property of fluorescein and its derivatives for the usefulness of the tracer compounds is the fluorescence of fluorescein. Fluorescein exists in two tautomeric forms, illustrated in Figure 4, depending on the acid concentration (pH) of the environment. In the open (acid) form, there are a number of conjugated double bonds which make that form of fluorescein (and compounds containing a fluorescein moiety) capable of absorbing blue light and emitting green fluorescence after an excited state lifetime of about four nanoseconds. When the open and closed forms coexist, the relative concentration of molecules in the open and closed forms is easily altered by adjustment of the pH level. Generally, the tracer compounds of the present invention exist in solution as biologically acceptable salts such as sodium, potassium or ammonium, which allows the compounds to exist in the open, fluorescent form, when employed in the analytical methods of the present invention. The specific salt present will depend on the buffer employed to adjust the pH level. For example, in the presence of a sodium phosphate buffer, the compounds of the present invention will generally exist in the open form, as a sodium salt.

As used herein, the term "fluorescein," either as an individual compound or as a component of a larger compound, is meant to include both the open and closed forms, if they exist for a particular molecule, except in the context of fluorescence. An open form is necessary for the fluorescence to occur.

The numbering of carbon atoms of the fluorescein molecule varies, depending upon whether the open or closed form of the molecule is considered. Accordingly, the literature concerning fluorescein and its compounds is not uniform as to carbon atom numbering. In the closed form, the para-carbon to the carbonyl of the lactone on the phenyl ring is numbered 6. In the open form, the para-carbon to the carboxylic acid group on the phenyl ring is numbered 5 (See Figure 3). In this disclosure the numbering of the closed form is adopted because the raw materials used in the syntheses are normally numbered with that system. The carbon atom of fluorescein and its compounds which is opposite the carboxyl group is therefore numbered "6" for the purpose of the present disclosure.

A tracer in solution which is not complexed to an antibody is free to rotate in less than the time required for absorption and re-emission of fluorescent light. As a result, the re-emitted light is relatively randomly oriented so that the fluorescence polarization of a tracer not complexed to an antibody is low, approaching zero. Upon complexing with a specific antibody, the tracer-antibody complex thus formed assumes the rotation of the antibody molecule which is slower than that of the relatively small tracer molecule, thereby increasing the polarization observed. Therefore, when a ligand competes with the tracer for antibody sites, the observed polarization of fluorescence of the resulting mixture of the free tracer and tracer-antibody complex assumes a value intermediate between that of the tracer and that of the tracer-antibody complex. If a sample contains a high concentration of the ligand, the observed polarization value is closer to that of the free ligand, i.e. low. If the test sample contains a low concentration of the ligand, the polarization value is

closer to that of the bound ligand, i.e. high. By sequentially exciting the reaction mixture of an immunoassay with vertically and then horizontally polarized light and analyzing only the vertically polarized component of the emitted light, the polarization of fluorescence in the reaction mixture may be accurately determined. The precise relationship between polarization and concentration of the ligand to be determined is established by measuring the polarization values of calibrators with known concentrations. The concentration of the ligand can be interpolated from a standard curve prepared in this manner.

The particular antibodies and tracers formed in accordance with this invention have been found to produce very good assays, as discussed infra.

1. The Reagents

Both the immunogens and the tracers of the present invention can be represented by the general structural formula set forth in the Summary of the Invention, and illustrated in Figure 2.

The objective is to have competition between ethosuximide and the tracer for the recognition sites of the antibody. Great variations in the structure of the haptens and tracers are allowed in achieving this goal. For the purpose of this invention, "haptens" are precursors of the immunogens, comprising generally a substituted succinimide derivative bearing a group suitable for linking to an immunologically active carrier.

a. The Structure of the Immunogens

Usable antibodies can be produced from a variety of succinimide derivatives. Immunogens made from compounds functionalized either at the 1 or 3 position on the ring can produce antibodies in animals; such antibodies are useful in an ethosuximide assay according to the invention when combined with the appropriate tracer.

The immunogens of the present invention have the general structural formula shown in Figure 2, and in the preferred form of the invention, the immunogens are also derived from the general structural formula shown in Figure 2. The immunogens can be prepared by coupling a compound of the class shown in Figure 2 with a poly (amino acid) or a derivative of a poly (amino acid) or other immunologically active carrier as will be discussed in the context of the synthetic method and the Examples below.

In a preferred form of the invention, the immunogen has the structural formula shown in Figure 2. Although substitution at either the 1 or 3 positions is equivalently preferred from a structural point of view, starting materials are more readily available and the synthesis is greatly simplified for derivatives of the 1-position on the ring. However, the probability of obtaining useful antisera from a given animal appears to be greater for derivatives of the 3-position. Although bovine serum albumin is the poly (amino acid) in this preferred form, it should be understood that various protein carriers can be employed, including albumins, serum proteins, e.g., globulins, ocular lens proteins, lipoproteins . Illustrative protein carriers include bovine serum albumin, keyhole limpet hemocyanin, egg ovalbumin, bovine gamma-globulin and thyroxine binding globulin. Alternatively, synthetic poly (amino acids) having a sufficient number of available carboxylate groups such as aspartates can be employed, as can many other synthetic or natural polymeric materials bearing reactive functional groups. In addition, carbohydrates, yeasts, polysacharides or any other substance that can be used as an immunological carrier can be conjugated to the hapten to produce an immunogen.

b. The Structure of the Tracers

The possible variations in the structure of the tracers of the invention are even greater than the possible variations in the structure of the haptens thereof. The tracers of the present invention have the general structural formula shown in Figure 2. In a preferred form of the invention, the tracer has the structural formula shown in Figure 5.

The tracer is an ethosuximide derivative that is linked to a fluorescein derivative by, for example, an amido, amidino, triazinylamino, carbamido, thiocarbamido, carbamoyl, thiocarbamoyl, or sulfonylcarbamoyl group, as shown in Figure 4. The tracers are prepared by linking the appropriate fluorescein derivative to an ethosuximide derivative containing for example an amino, carboxylic acid, sulfonic acid, mercapto, hydroxy, imidate, hydrazide, isocyanate, thioisocyanate, chloroformate, chlorothioformate, chlorosulfonylcarbamoyl group, as will be discussed in the context of the synthetic method and the Examples below.

By way of example, any of the following fluorescein derivatives can be used:

| | |
|---|---|
| Fl-$NH_2$ | fluorescein amine |
| Fl-$CO_2$H | carboxyfluorescein |
| Fl-$NHCOCH_2I$ | α-iodoacetamidofluorescein |
| Fl-$NHCOCH_2Br$ | α-bromoacetamidofluorescein |
| 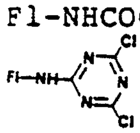 | (DTAF) 2, 4-dichloro-1,3,5,-triazin-2-ylamino-fluorescein |
| 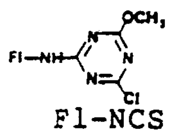 | 4-chloro-6-methoxy-1,3,5-triazin-2-ylamino-fluorescein |
| Fl-NCS | fluorescein thioisocyanate |

2. The Antibodies

The antibodies of the present invention are prepared by eliciting a response in rabbits to the immunogens described supra. The immunogen is administered to animals or to in vitro cultures of immunocompetent cells by a series of inoculations, in a manner well known to those skilled in the art. It should be understood that although rabbits were the preferred immune host to ethosuximide immunogens in the experiments detailed herein, any in vivo or in vitro host capable of producing antibodies to the structures herein outlined may be employed.

In reference to the drawings, $R_1$ may be H or -R-Z: when $R_1$ is R-Z, $R_2$ is methyl or ethyl; when $R_1$ is H, $R_2$ is R-Z; $R_3$ is methyl or ethyl. The orientation of $R_2$ and $R_3$ in the figure is not meant to imply nor to exclude any particular enantiomer. R is a group consisting of from 0 to 8 carbon atoms arranged in a straight or branched chain or in a phenylene or alkylenephenylene moiety; and Z is $NH_2$, $NHR_3$, Cl, Br, I, OH or CHO when the preparation is directed toward an immunogen and $NH_2$, COOH, CN, $SO_2Cl$, SH or OH when the preparation is directed toward a tracer.

a. The Synthesis of the Immunogens

The immunogens of the present invention are made by coupling a hapten, such as that shown by the general structure of Figure 2 when X is $NH_2$, chlorine, bromine, iodine or OH, to a poly (amino acid) or other immunologically active carrier. The poly (amino acid) or other carrier moiety can be linked to the hapten by an amide, a thioether, an ether, or a diazo linkage. In a preferred embodiment, the poly (amino acid), is bovine serum albumin (BSA), and the hapten has the structure shown in Figure 21. These reactants are preferably coupled under conditions normally used to form amide linkages; such conditions are well known to those skilled in the art. It is most preferred that carbodiimide procedures be used, as these are the most effective for forming the desired amide linkages in this context.

The immunogens are prepared by coupling a hapten that contains an -$NH_2$, -$CONHNH_2$, -Cl , -Br, -I, or -OH group to a poly (amino acid) or other immunologically active carrier. The -$NH_2$ case can be coupled by forming a diazonium salt and adding this to the poly (amino acid) or by activating the carboxylic acid group on the polyamino acid in the presence of the -$NH_2$ group. The diazonium salt method works only for aromatic amines and is typically prepared by mixing the amine with sodium nitrite in acid solution. The activation of the carboxylic acid groups on the poly (amino acid) can be accomplished by mixing the hapten and the poly (amino acid) with 1-ethyl-3-(3-dimethylamino-propyl) carbodiimide (EDC), N,N'-dicyclohexylcarbodiimide (DCC), 1-cyclohexyl-3-(2-morpholinoethyl) carbodiimide or methyl-p-toluene sulfonate. The -$CONHNH_2$ case is coupled in the same manner as for the non-aromatic amino case. Alkyl chloro, bromo, and iodo-derivatives and sulfonate esters alkylate the phenolic hydroxyl groups of tyrosine residues in the carrier protein under strongly alkaline conditions to form alkyl aryl ethers and alkylate the sulfur of free sulfhydryl groups cysteine to form thioethers. For these reactions the preferred derivatives are the iodides and sulfonate esters. The sulfonate esters are preferably produced from the alcohol derivatives by reaction with sulfonic acid halides or anhydrides in the presence of a tertiary amine base.

The synthesis of the above haptens (immunogen precursors) are accomplished in one of two general ways. Figure 2 shows an immunogen precursor class in accordance with a preferred embodiment of the method of the present invention. The preparation of 1-position derivatives proceeds from the appropriate

disubstituted succinic acid and the chosen linking arm bearing a primary amine on one end and the desired functional group (or a protected or latent form of it) at the other. The cyclization can be accomplished by treating a mixture of the reactants in solution with a dehydrating reagent such as N,N'-dicyclohexylcarbodiimide (DCC), or simply by heating an intimate mixture of the reactants to a temperature greater than about 100° C but less than the temperature at which severe pyrolytic degradation of reactants and products begins to take place. Latent or protected functionality is then revealed and the compound is coupled to the poly (amino acid) or other carrier.

In the preparation of 3-position derivatives, the Knoevenagel reaction method is preferred. The appropriate ketone and malonic acid or a derivative of it are condensed in the presence of a base, such as piperidine, or morpholine, which results in the formation of an $\alpha,\beta$-unsaturated carboxylic acid, dinitrile, diester or nitrile ester. This carboxylic acid derivative is reacted with alkali metal cyanide and then hydrolysed, which results in elimination of carbon dioxide and gives a substituted succinic acid which can be cyclized to the corresponding succinimide in the presence of ammonia. Latent or protected functionality is then revealed and the compound is coupled to a carrier.

Aromatic derivatives at the 1-position are prepared from the appropriate aniline or aralkyl amine. The alcohols at the 1-position are prepared from the requisite amino alcohol. The corresponding carboxylic acids are obtained from these by oxidation with a chromium-based oxidant such as Jones' Reagent.

The hydrazide derivatives are prepared from the carboxylic acids by active ester coupling with hydrazine salts.

Aromatic amines can be prepared from the appropriate aromatic compounds, by nitration, either before or after cyclization of the succinimide ring, and reduction, preferably by catalytic hydrogenation or by chemical reducing, agents such as sodium dithionite

Amines can be prepared from the alcohol having more than three atoms separating the hydroxyl group from the succinimide ring by converting the alcohol to a halide as described infra or to a sulfonate ester such as methanesulfonate or toluenesulfonate, displacing this with an azide ion from an alkali metal or quaternary ammonium azide and reducing the resulting azido group by catalytic hydrogenation or a chemical reducing agent. Amines having two or three atoms separating the amino group from the succinimide ring are preferably prepared by cyclizing an appropriate succinic acid onto a diamine in which one of the amino groups occurs in a protected or latent form such as dibenzylamino, followed by deprotection or revelation of the amine by reagents compatible with the succinimide ring structure such as catalytic hydrogenation. Amines may also be produced from aldehydes by reductive amination with ammonium acetate and sodium cyanoborohydride. These amine derivatives are then suitable for coupling to carriers to produce immunogens.

The alkyl halides may be prepared by treatment of the alcohols with hydrogen chloride, hydrogen bromide or hydrogen iodide or by treatment with halogenating reagents such as phosphorus pentachloride, phosphorus tribromide or thionyl chloride. These halides couple directly to free sulfhydryl groups on carriers under relatively neutral conditions or to phenols, such as those of tyrosyl residues in a poly(amino acid), under strongly basic conditions.

Many ethosuximide derivatives attached through the 3-position on the ring are prepared preferably by employing a terminal olefin as latent functionality. In this preferred embodiment, a methyl omega-alkenyl or ethyl omega-alkenyl ketone is converted to a succinimide by the Knoevenagel pathway described above. The terminal olefin is then most preferably subjected to ozonolysis followed by mild reduction employing dimethylsulfide or catalytic hydrogenation to give an aldehyde, or to more vigorous reduction with a borohydride reducing agent such as sodium borohydride to give a terminal alcohol, or to oxidation with a chromium-based oxidizer such as Jones' Reagent to give the corresponding carboxylic acid.

b. The Synthesis of the Tracers

The tracers of the present invention are made by coupling a fluorescein moiety, or a derivative of fluorescein, to the general structure shown in Figure 2 wherein $R_1$ is H or -R-Y; when $R_1$ is -R-Y, $R_2$ is methyl or ethyl; when $R_1$ is H, $R_2$ is $CH_2$-R-Y; $R_3$ is methyl or ethyl; R is a group consisting of from 0 to 8 carbon atoms arranged as a straight or branched chain or in a phenylene or alkylenephenylene moiety; and Y is $NH_2$, $SO_3H$, COOH, CN, $SO_2Cl$, CHO, SH or OH. The fluorescein moiety can be linked to the amino, carboxyl, imidate or alkoxy functional group by an amide, an amidine, a urea, a thiourea, a carbamate, a thiocarbamate, triazinylamino or sulfonylcarbamate linkage, as shown in Figure 4. In the presently preferred embodiment, the fluorescein derivative is 6-carboxyfluorescein (available from Calbiochem, La Jolla, California) and this is coupled to a precursor shown in Figure 2. [$R_1$ = H, $R_3$ = methyl, $R_2$ = $(CH_2)_3NH_2$]. The 6-carboxyfluorescein is coupled to 3-methyl-3-aminopropyl succinimide by first forming the active ester of

6-carboxyfluorescein. The preferred active ester is N-hydroxysuccinimide active ester and the preferred method is via N,N'-dicyclohexylcarbodiimide activation in dry pyridine. Other activating groups, such as 1-hydroxybenzotriazole, p-nitrophenol, and carbonyldiimidazole, can be used; and other solvents, such as dimethylformamide and dimethylsulfoxide, can be used. The reactants are preferably coupled under conditions for forming amide linkages, and it is most preferred that active ester procedures be used. Usable tracers can be prepared from a variety of ethosuximide derivatives.

All ethosuximide derivatives that have a terminal amino group, such as amino, hydrazinyl, hydrazido, are coupled to carboxyfluorescein by the active ester method or the mixed anhydride method, and coupled to fluorescein isothiocyanate, DTAF or alkoxy DTAF by simply mixing the two materials in solution. The amino group can be converted to the isocyanate and thioisocyanate groups by reaction with phosgene and thiophosgene, respectively. These are then condensed with aminofluorescein to produce the tracer.

All ethosuximide derivatives that have a terminal carboxylic acid group, such as carboxylic acid, (aminohydroxy)alkylcarboxylic acid are coupled to aminofluorescein by the active ester method.

All ethosuximide derivatives that have a terminal hydroxy group can be coupled to fluorescein by reaction with DTAF, α-iodoacetamidofluorescein, α-bromoacetamidofluorescein or fluorescein isothiocyanate in solution. The hydroxy group can be converted to the chlorosulfonylcarbamoyl, chloroformate and chlorothioformate groups by reaction with chlorosulfonylisocyanate, phosgene and thiophosgene, respectively. These derivatives are then coupled to aminofluorescein in solution to produce the tracer.

Ethosuximide derivatives having a terminal mercapto group are prepared from halides or sulfonate esters and alkali metal sulfides as described supra for azides. They are coupled in solution with α-iodoacetamidofluorescein or α-bromoacetamidofluorescein.

Ethosuximide derivatives that have a terminal nitrile group can be prepared from halides or sulfonate esters as described supra for azides. They are converted to imidates in anhydrous alcohol in the presence of hydrogen chloride gas. The imidate is then coupled to fluorescein amine in solution to prepare the tracer.

Preparation of the various amino, hydroxy and aromatic derivatives of the ethosuximide derivatives has been described above in the immunogen preparation sections.

The sulfonic acids are produced preferably from the appropriate aminoalkylsulfonic acid, such as taurine, by cyclizing under the conditions described supra. They are converted to the corresponding sulfonyl chlorides by reaction with a chlorinating reagent such as thionyl chloride phosphoryl chloride or phosphorus pentachloride. Direct introduction of sulfonyl chloride groups into aromatic rings is accomplished by treatment with chlorosulfuric acid. The sulfonyl halides are then reacted with amino fluoresceins or derivatives of fluorescein bearing reactive amino groups, usually with additions of a tertiary amine base.

3. The Assay

The particular tracers and antibodies of the present invention have been found to accurately and specifically measure levels of ethosuximide in serum and plasma. Figure 1 shows the structure of the drug ethosuximide that can be quantitatively determined in accordance with the present invention. The assay of the present invention provides a more rapid and convenient ethosuximide assay method than prior art methods, because it requires no specimen treatment before analysis, the reagents are chemically and thermally stable and the assay system has minimal cross-reactivity to ethosuximide-like compounds.

In accordance with the analytical methods of the present invention, i.e., the methods of determining ethosuximide by a fluorescence immunoassay procedure using the tracer compounds and immunogens of the invention, a sample containing or suspected of containing ethosuximide is intermixed with a biologically acceptable salt of a tracer and an antibody specific to ethosuximide and the tracer. The antibody is produced using the immunogen as described above. The ethosuximide and tracer compete for limited antibody sites, resulting in the formation of complexes. By maintaining constant the concentration of tracer and antibody, the ratio of ethosuximide antibody complex to tracer-antibody complex that is formed is directly proportional to the amount of ethosuximide in the sample. Therefore, upon exciting the mixture with linearly polarized light and measuring the polarization of the fluorescence emitted by a tracer and a tracer-antibody complex, one is able to determine quantitatively the amount of ethosuximide in the sample.

The results can be quantified in terms of net millipolarization units, span (in millipolarization units) and relative intensity. The measurement of millipolarization units indicates the maximum polarization when a maximum amount of the tracer is bound to the antibody in the absence of any ethosuximide. The higher the net millipolarization units, the better the binding of the tracer to the antibody. The span is an indication of the difference between the net millipolarizations at the points of maximum and minimum amount of tracer bound to the antibody. A larger span provides for a better numerical analysis of data. The intensity is a measure of the strength of the signal above background. Thus, a higher intensity will give a more accurate

measurement. The intensity is determined at about 0.5 to 2.0 nanomolar for the preferred tracers of the invention, as the sum of the vertically polarized intensity plus twice the horizontally polarized intensity. The intensity of the tracer signal can range from about three times to about thirty times the background noise, depending upon the concentration of the tracer and other assay variables. For the purposes of the present invention, an intensity of at least eight to ten times that of background noise is preferred.

Tables I through III show the results obtained with various embodiments of the present invention, in terms of span, millipolarization units and intensity. In all instances, bovine serum albumin (BSA) was used as the protein carrier. Table I shows data for assays produced by pairing antiserum raised against one of the novel immunogens of this invention with several of the tracers described herein. Table II shows that the 3-position tracers of this invention can also be employed with appropriate antisera raised against immunogens of the prior art. Finally, Table III demonstrates that tracers of this invention which are derivatives of the 1-position of the succinimide are also useful when paired with appropriate antisera. As seen from these data, an assay produced from an immunogen made from the hapten of Figure 21 used in combination with the tracer of Figure 5 provides excellent results. Accordingly, this combination is presently the most preferred form of the invention. In addition, the hapten/tracer combinations represented by the combinations of Figures 21 and 8, Figures 21 and 7, Figures 22 and 7 and Figures 23 and 5 also produced acceptable results and are alternative preferred combinations.

Table I

| Assays with the novel hapten of Figure 21 | | | |
|---|---|---|---|
| Tracer | Net Polarization* | Span* | Intensity** |
| Fig. 5 | 202 | 153 | 18 |
| Fig. 7 | 197 | 140 | 13 |
| Fig. 8 | 203 | 148 | 17 |

*In millipolarization units.
**Expressed as a ratio of net intensity to background noise.

Table II

| Assays with 3-position Prior Art immunogens | | | | |
|---|---|---|---|---|
| Hapten | Tracer | Net Polarization* | Span* | Intensity** |
| Fig. 22 | Fig. 5 | 220 | 128 | 18 |
| Fig. 22 | Fig. 6 | 185 | 40 | 17 |
| Fig. 22 | Fig. 7 | 210 | 153 | 13 |
| Fig. 22 | Fig. 8 | 252 | 110 | 17 |
| Fig. 23 | Fig. 5 | 197 | 148 | 18 |

*In millipolarization units
**Expressed as the ratio of net intensity to background noise

Table III

| Assays with 1-position Prior Art immunogens prepared from the hapten of Fig. 24 | | | |
|---|---|---|---|
| Tracer | Net Polarization* | Span* | Intensity** |
| Fig. 9 | 187 | 107 | 7 |
| Fig. 10 | 155 | 107 | 5 |
| Fig. 11 | 186 | 74 | 11 |
| Fig. 12 | 268 | 57 | 8 |
| Fig. 13 | 198 | 56 | 15 |
| Fig. 14 | 292 | 51 | 24 |
| Fig. 15 | 210 | 51 | 5 |
| Fig. 16 | 173 | 47 | 11 |
| Fig. 17 | 252 | 39 | 13 |
| Fig. 18 | 190 | 36 | 16 |
| Fig. 19 | 232 | 38 | 3 |
| Fig. 20 | 214 | 37 | 6 |

*In millipolarization units.
**Expressed as the ratio of net intensity to background noise

The pH at which the method of the present invention is practiced must be sufficient to allow the fluorescein moiety of the tracers to exist in their open form. The pH may range from about 3 to 12, more preferably in the range of from about 5 to 10, and most desirably from about 6 to 9. Various buffers may be used to achieve and maintain the pH during the assay procedure. Representative buffers include borate, citrate, acetate phosphate, carbonate, tris or barbital. The particular buffer employed is not critical to the present invention, but the tris and phosphate buffers are preferred. The cation portion of the buffer will generally determine the cation portion of the tracer salt in solution.

The preferred method of the improved assay of the present invention will now be discussed in detail. The assay is a "homogeneous assay," which means that the end polarization readings are taken from a solution in which bound tracer is not separated from unbound tracer. This is a distinct advantage over heterogeneous immunoassay procedures such as those where the bound tracer must be separated from the unbound tracer before a reading can be taken.

The reagents for the fluorescence polarization assay of the present invention comprise antibody specific for ethosuximide and tracer. Additionally, largely conventional solutions including a ethosuximide pretreatment solution, a dilution buffer, ethosuximide calibrators and ethosuximide controls are desirably prepared. Typical solutions of these reagents, some of which are described herein, are commercially available in assay "kits" from Abbott Laboratories, Abbot Park, Illinois.

All percentages expressed herein are weight/volume unless otherwise indicated. The tracer formulation presently preferred is 70 nanomolar tracer in: 0.1 molar tris buffer at pH 5.5; 5% 5-sulfosalicylate; and 0.1% sodium azide. The antiserum formulation comprises rabbit serum diluted with: 0.1 molar tris buffer at pH 7.5; 0.1%c sodium azide; 0.1% bovine gamma globulin; and 2% ethylene glycol (volume/volume). The dilution buffer comprises: 0.1 molar sodium phosphate at pH 7.5; 0.1% sodium azide; and 0.01% bovine gamma globulin. The pretreatment formulation comprises: 0.1 molar tris buffer at pH 5.5; 5% 5-sulfosalicylate; 0.1% sodium azide. Calibrators comprising ethosuximide in normal human serum at concentrations of 0.0, 10, 25, 50, 100 and 150 milligrams per liter, with 0.1% sodium azide preservative are useful. Controls comprising ethosuximide in normal human serum are provided at concentrations of 35, 70 and 120 milligrams per liter with 0.1% sodium azide as a preservative.

The preferred procedure is especialy designed to be used in conjunction with the Abbott TDx® Polarization Analyzer available from Abbott Laboratories, Irving, Texas. 50 Microliters of serum or plasma are required. The calibrators, controls, or unknown samples are pipetted directly into the sample well of the TDx® sample cartridge. One of the advantages of this procedure is that the sample does not require any special preparation. If a TDx® ethosuximide assayn kit is being used with the TDx® analyzer, samples are placed directly into a sample carousel, the caps from each of the three reagent containers in the kit are removed and placed into designated wells inside the TDx® analyzer, and the assay procedure from this point is fully automated.

If a manual assay is being performed, then the sample is mixed with the pretreatment solution in dilution buffer and a background reading is taken. The tracer is then mixed with the assay. The antibody is

then finally mixed into the test solution. After incubation, a fluorescence polarization reading is taken.

The fluorescence polarization value of each calibrator, control or sample is determined and is printed on the output tape of an instrument such as the Abbott TDx® polarization analyzer. A standard curve is generated in the instrument by plotting the polarization of each calibrator versus its concentration using nonlinear regression analysis. The concentration of each control or sample is read off the stored calibration curve and printed on the output tape.

With respect to the foregoing preferred procedure, it should be noted that the tracer, antibody, pretreatment solution, calibrators and controls should be stored between about 2 and about 8 degrees C, while the dilution buffer should be stored at ambient temperature. A standard curve and controls should be run every two weeks, with each calibrator and control run in duplicate. Controls should be run daily and all samples can be run in replicates if so desired.

It should be understood that the foregoing detailed description and the following Examples are intended to be illustrative, but not limiting, with respect to the scope of the present invention. Various modifications will become apparent to one skilled in the art, and thus it is intended that the scope of the invention be defined solely by the claims and legal equivalents thereof.

## EXAMPLES

Examples I through XLV describe experiments that were performed in accordance with the concepts of the present invention. Example I is directed to preparation of an immunogen useful for producing antibody; Examples II through XXIX are directed to the synthesis of precursors for immunogens and tracers; and Examples XXX through XLV are directed to the preparation of tracers.

### Example I

### Preparation of an Immunogen

The Immunogen for the preferred assay was made by dissolving 29 milligrams of the hydrochloride of the compound illustrated in Figure 21 in 2 ml. of 0.1 M acetate buffer (pH 5.0) containing 29 mg. of bovine serum albumin. While the solution was stirred at room temperature, 140 mg. of 1-ethyl-3-(3'-dimethylaminopropyl) carbodiimide was added in 10 to 15 mg. portions over a period of 3.5 hours, maintaining the pH between 5.0 and 5.5. The resulting solution was dialyzed exhaustively versus 0.9% saline and then centrifuged at 10000 rpm for 30 minutes. The solution was stored frozen until used for immunizations.

### Example II

### 3-Methyl-3-(3-aminopropyl)succinimide hydrochloride

6.58 g. Of 5-chloro-2-pentanone ethylene ketal and 7.88 g. of dibenzylamine were heated under nitrogen atmosphere in an oil bath at 130° C for 38 hours. The cooled residue was taken up in chloroform and washed with 2 M sodium hydroxide solution. After removal of solvent, the residue was again heated under nitrogen for 24 hours. After a second base wash to remove HCl, the residue was heated for a third time for another 48 hours. After a final base wash, the crude product was chromatographed over 550 g. Silica gel with hexane-dichloromethane-ethyl acetate to give 8.8 g 5-dibenzylamino-2-pentanone ethylene ketal as a nearly colorless oil. 6.54 g. Of this ketal were dissolved in 20 ml. of tetrahydrofuran and 16 ml. of 1.5 M hydrochloric acid was added. After allowing the reaction to proceed for 4 hours at room temperature, the mixture was partitioned between ether and dilute HCl. The aqueous layer was chilled, made basic with solid potassium hydroxide and extracted into dichloromethane. The residue after drying and removal of solvent was 5.5 g. of 5-dibenzylamino-2-pentanone, a colorless oil. 2.81 g. of this ketone were dissolved in 30 ml. of benzene and 1.24 g. of ethyl cyanoacetate, 0.30 g. acetic acid, 0.11 g. of ammonium acetate, and 0.12 g. of piperazine were added. The mixture was heated under reflux for 24 hours, removing the water liberated with a Dean-Stark trap. The cooled solution was extracted four times with saturated potassium carbonate solution, dried over solid potassium carbonate and filtered through 5 g. of silica gel, rinsing with additional benzene. Evaporation of the solvent left 3.24 g. of nearly pure ethyl 2-cyano-6-dibenzylamino-3-methyl-2-hexenoate. 2.26g of this unsaturated cyanoester were dissolved in 9 ml. Of ethanol plus 3 ml. Of distilled water, and 0.35 g. of sodium cyamide were added. After stirring at room temperature for 40 minutes, the mixture was heated in an oil bath at 50 degrees for 30 minutes. The solvents were removed,

and the residue was taken up in dichloromethane, filtered, and the solvent removed to leave 2.28 g. of crystalline solid. 2 g. Of this material was taken up in 10 ml. of 10% sodium hydroxide solution and heated under reflux for 15 hours. The reaction mixture was cooled, acidified to pH2 2.5 with HCl and concentrated to dryness. The residue was taken up in 50 ml. of absolute methanol and filtered to remove salts. Concentration of the filtrate left 2.6 g. of a solid, of which 2.5 g. were subsequently dissolved in 15 ml. Of methanol and 15 ml. of concentrated aqueous ammonia. The solvents were distilled off and the residue was heated in an oil bath at 165° C for 30 minutes. The crude product was purified by preparative thin layer chromatography with chloroform containing 5% methanol to give 0.48 g. of 3-methyl-3-(3-dibenzylamine-opropyl) succinimide. The benzyl protecting groups were removed by hydrogenation of 0.35 g. of this material in 50 ml. of ethanol plus 85 ml. of concentrated HCl over 0.07 g. of 10% palladium on charcoal at an initial pressure of 3 atmospheres. After four days the catalyst was filtered off and the solvent removed to give 0.25 g. of 3-methyl-3-(3-aminopropyl)succinimide hydrochloride as a white solid.

Example III

3-Methyl-3-(3-butenyl)succinimide

24.5 g. Of 1-hexene-5-1 and 28.25 g. of ethyl cyanoacetate were dissolved in 25 ml. of benzene. Acetic acid (2.86 ml.) and ammonium acetate (1.93 g.) were added and the mixture was heated under reflux for 6 hours, collecting the water liberated in a Dean-Stark trap. The cooled solution was transferred to a separatory funnel and washed twice with water. Solvent was removed in vacuo. The residue was dissolved in a mixture of 50 ml. water and 50 ml. ethanol, cooled in an ice bath, and treated with 12.5 g. sodium cyanide. After 15 minutes the ice bath was removed and the reaction was allowed to proceed for 2 hours at room temperature. After removing most of the ethanol on a rotary evaporator, the pH of the mixture was adjusted to 5 with HCl. The solution was rinsed three times with ether and the pH was lowered to about 1. Three ether extracts were combined, dried with magnesium sulfate, filtered and concentrated in vacuo to give 37.8 g. of a golden oil. One gram of this material was taken up in 2 ml. of 2 M sodium hydroxide solution and heated in a 90° C oil bath for four hours. Another 1 ml. of 2 M sodium hydroxide was added and heating was resumed for a further 2 hours. All material was brought into solution in 2 to 1 water-methanol and the pH was adjusted to 3 with HCl. The solvents were removed, and the residue was triturated with absolute methanol. After filtration, removal of solvent left 1.0 g. of a pale yellow oil. This oil was dissolved in 5 ml. concentrated aqueous ammonia, heated at 100° C until all solvent water had distilled off and then at 140 to 150° C for 3 hours. The cooled residue was chromatographed over silica gel with dichloromethane-ether to give 0.77 g. of white crystalline 3-methyl-3-(3-butenyl) succinimide, m.p. 75° C.

Example IV

3-Methyl-3-(4-hydroxybutyl)succinimide

3-Methyl-3-(3-butenyl)succinimide (3 g., 18 mmoles) was dissolved in 50 ml. ethanol, chilled in a dry ice - isopropanol bath and treated with one equivalent of ozone. Excess ozone was purged out with nitrogen and 684 mg. (18 mmoles) of sodium borohydride were added. The mixture was allowed to warm gradually to room temperature and quenched by addition of a few drops concentrated hydrochloric acid. The solvent was removed, and the residue redissolved in dichloromethane, dried over magnesium sulfate, treated with a little silica gel, filtered and again stripped down. The colorless oil which remained (2.80 g.) was identified as the alcohol by NMR and mass spectrometry.

Example V

3-Methyl-3-ethyl-1-(2-hydroxyethyl)succinimide

Ethanolamine (1.22 g., 20 mmoles) and 2-ethyl-2-methyl succinic acid (3.20 g., 20 mmoles) were mixed without solvent and heated in an oil bath, gradually raising the temperature from 120 to 160° C over the course of two hours. A distillation head was installed on the flask and the product distilled at 131 to 132° C under a pressure of 0.5 Torr to give 2.84 of a colorless oil.

Example VI

3-Methyl-3-ethyl-1-(3-hydroxy-1-propyl)succinimide

3-Amino-1-propanol (1.50 g., 20 mmoles) and 2-ethyl-2-methyl succinic acid (3.20 g., 20 mmoles) were mixed without solvent and heated in an oil bath, raising the temperature gradually from 120 to 160° C over the course of two hours. A distillation head was installed on the flask and the product was distilled to give 2.75 g. of a colorless oil boiling at 126 to 128° C under a pressure of 0.36 Torr.

Example VII

1-Dibenzylamino-3-phthalimidopropane

Dibenzylamine (2.96 g., 15 mmoles) and 3-bromopropylphthalimide (4.02 g., 15 mmoles) were taken up in 10 ml. of methanol plus 2 ml. of tetrahydrofuran and heated at 40 to 45° C for a total of approximately 30 hours, an equivalent (2.07 g.) of potassium carbonate having been added after 24 hours. The reaction mixture was partitioned between dichloromethane and 1 M aqueous potassium hydroxide; the organic layer was washed twice more with potassium hydroxide, dried over anhydrous potassium carbonate and concentrated. The semi-crystalline residue was recrystallized from chloroform-methanol to give a total of 2.46 g. of 1-dibenzylamino-3-phthalimidopropane, melting at 111.5 to 112.5° C.

Example VIII

1-Amino-3-dibenzylaminopropane

1-Dibenzylamino-3-phthalimidopropane (1.92 g., 5 mmoles) and 1.25 g. (25 mmoles) of hydrazine hydrate were dissolved on warming in 20 ml. of ethanol. As heating was continued to reflux temperature, a white crystalline solid was deposited. After one hour, the reaction mixture was cooled, the solid was dissolved in dilute sodium hydroxide and the majority of the ethanol was removed on a rotary evaporator. The solution was made strongly basic with solid potassium hydroxide and extracted with ether. The organic layer was washed with aqueous base, dried over anhydrous potassium carbonate, filtered and condensed to give 1.26 of a colorless oil.

Example IX

3-Methyl-3-ethyl-1-(3-dibenzylamino-1-propyl) succinimide hydrochloride

1-Amino-3-dibenzylaminopropane (1.02 g., 4 mmoles) and 2-ethyl-2-methyl succinic acid (0.64 g., 4 mmoles) were mixed without solvent and heated in an oil bath at 140° C for 1 1/2 hours. The cooled crude product was dissolved in a mixture of dichloromethane and ethyl acetate treated with a small amount of silica gel to decolorize, filtered and condensed to give 1.38 g. of a nearly colorless oil. Most of this material was redissolved in dichloromethane and treated with anhydrous hydrogen chloride. No crystallization took place from the solution, nor did the residue solidify upon removal of the solvent.

Example X

3-Methyl-3-ethyl-1-(3-aminopropyl)succinimide hydrochloride

3-Methyl-3-ethyl-1-(3-dibenzylaminopropyl) succinimide hydrochloride (1.01 g., 2.4 mmoles) was dissolved in 50 ml. anhydrous ethanol and hydrogenated over 10% palladium on charcoal at an initial pressure of 2 atmospheres. Catalyst was filtered off and replaced twice during the course of the reaction; total reaction time was approximately 48 hours. The product was 0.57 g. of a colorless oil.

Example XI

3-Methyl-3-ethyl-1-(4-hydroxy-1-butyl)succinimide

4-Amino-1-butanol (270 mg., 3 mmoles) and $\alpha$-methyl-sh $\alpha$-ethylsuccinic acid (480 mg., 3mmoles) were mixed without solvent and heated in an oil bath at 135 to 140° C for 3 1/2 hours. After cooling, the product was taken up in dichloromethane and decolorized by treatment with a little silica gel. Filtration and removal

of solvent left 537 mg. of a colorless oil.

Example XII

3-(3-Methyl-3-ethyl-1-succinimidyl)paropionic acid

3-Methyl-3-ethyl-1-(4-hydroxy-1-butyl)succinimide (213 mg., 1 mmole) was dissolved in 5 ml. of acetone and 0.45 ml. of a 2.67 M solution of Jones' reagent (1.2 mmole) was added. After stirring at room temperature for 2 hours, a few drops of isopropanol were added to consume excess reagent. After stirring for 15 minutes more, the solution was diluted with 5 ml. dichloromethane, treated with magnesium sulfate and filtered through Celite®. Removal of solvent left 207 mg. of the acid as a colorless oil.

Example XIII

3,3-Dimethyl-1-(5-hydroxy-1-pentyl)succinimide

5-Amino-1-pentanol (310 mg., 3 mmoles) and 2,2-dimethylsuccinic acid were mixed and heated neat in an oil bath at 125 to 135° C for 4 hours. The cooled residue was dissolved in dichloromethane, decolorized with a small amount of silica gel and filtered. Removal of solvent left 569 mg. of 3,3-dimethyl-1-(5-hydroxy pentyl)succinimide as a clear, colorless oil.

Example XIV

3,3-Dimethyl-1-(4-carboxybutyl)succinimide

3,3-Dimethyl-1-(5-hydroxy-1-pentyl)succinimide (236 mg., 1.11 mmole) was dissolved in 5 ml. Of acetone and 520 ml. of a 2.67 M solution of Jones' Reagent was added. The mixture was stirred at room temperature for 2 hours, and 4 drops of isopropanol were added to quench excess reagent. After stirring for 15 minutes more, the solution was diluted with 5 ml. of dichloromethane, treated with magnesium sulfate, filtered and concentrated to give 250 mg. of the acid as a colorless oil.

Example XV

1-(6-Hydroxyhexyl)-3-methyl-3-ethylsuccinimide

6-Amino-1-hexanol (586 mg., 5 mmoles) and 801 mg. of α-methyl-α-ethylsuccinic acid (5 mmoles) were mixed without solvent and heated in a 130° C oil bath for 3 hours. After cooling to room temperature, the residue was taken up in dichloromethane, treated with a little silica gel to decolorize the solution and filtered. The residue upon evaporation of the solvent was 959 mg. of 1-(6-hydroxyhexyl)-3-methyl-3-ethyl succinimide, a colorless oil.

Example XVI

1-(5-carboxypentyl)-3-methyl-3-ethylsuccinimide

1-(6-Hydroxyhexyl)-3-methyl-3-ethyl succinimide (705 mg., 2.9 mmoles) was dissolved in 15 ml. of acetone and 2.0 ml. of a 2.67 M solution of Jones' Reagent (3.3 mmoles) was added. After stirring at room temperature for 2 hours, a few drops of isopropanol were added to destroy excess reagent. The mixture was diluted with an equal volume of dichloromethane, treated with magnesium sulfate and filtered through Celite®. The residue upon evaporation of solvents was 735 mg. of the acid as an oil.

Example XVII

3-Methyl-3-ethyl-(5-Hydroxypentyl)succinimide

This compound was prepared from 4.12 g. of 5-amino-1-propanol (40 mmole) and 6.40 g. of 2-ethyl-2-methylsuccinic acid by the procedure of Example XV. The yield was 8.48 of a nearly colorless oil.

Example XVIII

3-Methyl-3-ethyl-1-(4-carboxybutyl)succinimide

A solution of 908 mg. of 3-methyl-3-ethyl-1-(5-hydroxypentyl)succinimide in 20 ml. acetone was treated at room temperature with 1.5 ml. of a 2.67 M solution of Jones' Reagent. Since the reaction did not go to completion after a reasonable interval, a further 0.45 ml. of Jones' Reagent solution was added. One hour later, the excess was quenched by addition of 5 drops of isopropanol. The mixture was filtered through Celite® and solvent was removed. The residue was taken up in dichloromethane, treated with magnesium sulfate, filtered again and solvent was removed to leave 978 mg. of the title compound as a colorless oil.

Example XIX

4-(3-Methyl-3-ethyl-1-succinimidyl)nitrobenzene

4-Nitroaniline (2.76 g., 20 mmoles) and α-methyl-α-ethylsuccinic acid were mixed intimately and heated in an oil bath at 150 to 160° C for 2 hours. The product, which solidifed on cooling, was recrystallised from methanol-water to give nearly white crystals melting at 124-125.5° C.

Example XX

4-(3-Methyl-3-ethyl-1-succinimidyl)aniline

4-(3-Methyl--ethyl-1-succinimidyl)nitrobenzene (500 mg., 1.91 mmoles) was hydrogenated in 50 ml. Of ethanol over 50 mg. of 10% palladium on carbon at an initial pressure of 2 atmospheres. After 20 minutes the catalyst was filtered off and the solvent removed, leaving ca. 500 mg. of a colorless oil which crystallized on standing. It melted at 140-143° C after recrystallization from methanol.

Example XXI

3-(3-Methyl-3-ethyl-1-succinimidyl)nitrobenzene

3-Nitroaniline (2.76 g., 20 mmoles) and α-methyl-α-ethylsuccinimide (3.20 g., 20 mmoles) were mixed well and heated without solvent in an oil bath at 150 to 160° C for 2 hours. The product, which solidified on cooling, was recrystallized from methanol to give a total of 3.72 g. of crystals from two crops, m.p. 87.5 to 88.5° C.

Example XXII

3-(3-Methyl-3-ethyl-1-succinimidyl)aniline

3-(3-Methyl-3-ethyl-1-succinimidyl)nitrobenzene (500 mg., 1.91 mmoles) was hydrogenated in 50 ml. of ethanol over 50 mg. of 10% palladium on carbon at an initial pressure of 2 atmospheres. After 30 minutes the catalyst was filtered off and the solvent removed to leave 480 mg. of a colorless oil which crystallized overnight. Recrystallization from methanol-water gave a colorless solid melting at 100 to 102° C.

Example XIII

1-Benzyl-3-methyl-3-ethyl succinimide

Benzylamine (2.14 g., 20 mmole) and α-methyl-α-ethylsuccinic acid (3.20 g., 20 mmole) were mixed without solvent and heated in an oil bath at 140° C for 2 hours. The material was cooled, dissolved in dichloromethane, treated with a little silica gel to decolorize and filtered. The residue upon removal of solvent was 4.32 g. of 1-benzyl-3-methyl-3-ethyl succinimide as a nearly colorless oil.

Example XXIV

4-(3-Methyl-3-ethyl-1-succinimidyl(methyl)benzene sulfonyl chloride

1-Benzyl-3-methyl-3-ethyl succinimide (924 mg., 4 mmoles) was dissolved in 3 ml. of chlorosulfuric acid. the initial exothermic reaction was allowed to subside, and the mixture was heated in a 40 °C bath for 15 minutes. It was poured over crushed ice and extracted with dichloromethane. The organic layer was washed twice with saturated brine, dried over magnesium sulfate and filtered through a small pad of silica gel. Removal of solvent left 926 mg. of the sulfonyl chloride as a colorless oil.

Example XXV

2-(3-Methyl-3-ethyl-1-succinimidyl)ethylbenzene

This compound was prepared from phenethylamine and α-methyl-α-ethylsuccinic acid essentially as in Examples XV and XXIII. The product was a nearly colorless oil.

Example XXVI

2-[2-(3-Methyl-3-ethyl-1-succinimidyl)ethyl] aniline

2-(3-Methyl-3-ethyl-1-succinimidyl) ethyl benzene (990 mg., 3.75 mmole) was dissolved in 4 ml. acetic anhydride. To this was added, with stirring, a mixture made from 0.5 ml. of concentrated nitric acid and 2 ml. of ice-chilled acetic anhydride. After 15 minutes at room temperature, the reaction was heated in a 50 °C bath for 1 hour. It was quenched by addition of water and volatile materials were removed. The residue was partitioned between water and ether, and the organic layer was washed with water, dried with magnesium sulfate, filtered and stripped to dryness. Approximately 1/3 of the crude product was chromatographed on preparative thin layer plates to give 350 mg. of an oily mixture of the para- and ortho-isomers, free of unreacted starting material. 300 mg. Of this mixture were hydrogenated in 75 ml. of ethanol over 50 mg. of 5% palladium on charcoal at an initial pressure of 2 atmospheres. The reaction was complete within 1 hour. The product mixture was separated by thin layer chromatography, developing with benzene-ethyl acetate, to give 120 mg. of para-isomer and 80 mg. of ortho-isomer.

Example XXVII

5-(3-ethyl-succinimid-1-yl)-1-pentyl methane sulfonate

5-(3-Methyl-3-ethyl-succinimid-1-yl)-1-pentanol (908 mg., 4 mmoles) and triethylamine (558 ml., 4 mmoles) were dissolved in 10 ml. of dichloromethane and chilled in an ice-water bath. Methane sulfonyl chloride (277ml. 4 mmoles) was added, and the mixture was stirred for 30 minutes removing the ice bath after 5 minutes. The reaction was incomplete, so a further 100 ml. of triethylamine and 45 ml. of methanesulfonyl chloride were added, and stirring was continued for 45 minutes more. The reaction mixture was partitioned between dilute HCl and dichloromethane; the organic layer was washed with 10% aqueous sodium bicarbonate, dried over magnesium sulfate and the solvent was removed. The mesylate was a colorless oil; yield was 1.15 g.

Example XXVIII

5-(3-Methyl-3-ethylsuccinimid-1-yl)-1-pentyl azide

5-(3-Methyl-3-ethylsuccinimid-1-yl)-1-pentyl methanesulfonate (0.92 g., 3 mmoles) and sodium azide (0.39 g., 6 mmoles) were heated and stirred in 5 ml. of dimethylformamide at 140 °C for 1 hour. The dimethylformamide was removed in vacuo, and the residue was taken up in dichloromethane, decolorized with a little silica gel and filtered through a further small amount of silica gel. Removal of solvent left 0.86 g. of a nearly colorless oil identified as the azide by NMR and mass spectrum.

Example XXIX

5-(3-Methyl-3-ethylsuccinimid-1-yl)-1-pentylamine hydrochloride

This compound is prepared by hydrogenation of 5-(3-methyl-3-ethylsuccinimid-1-yl)-1-pentyl azide in ethanol containing one equivalent of hydrochloric acid over a palladium on charcoal catalyst.

Example XXX

6-[3-(3-Methyl-3-succinimidyl)propyl amino carbonyl] fluorescein

6-Carboxyfluorescein (215 mg.) and 63 mg. of N-hydroxysuccinimide were dissolved in 5 ml. of anhydrous pyridine and 124 mg. of N,N'-dicyclohexylcarbodiimide was added. The mixture was stirred at room temperature for 2 hours and 103 mg. of 3-methyl-3-(3-aminopropyl)succinimide hydrochloride were added. The mixture was stirred at room temperature for 20 hours and the crude product was purified by preparative thin layer chromatography, twice with 3:1 chloroform-methanol and once with 1:1:1 benzene-ethyl acetate-acetone to give the tracer.

Example XXXI

5-[3-(3-Methyl-3-succinimidyl)propylaminocarbonyl] fluorescein

This compound was prepared according to the procedure of Example XXX, starting with 10 mg. of 5-carboxyfluorescein, 6.2 mg. of dicyclohexylcarbodiimide, 4.2 mg. of 1-hydroxybenzotriazole (rather than N-hydroxysuccinimide) and 5 mg. of 3-methyl-3-(3-aminopropyl) succinimide hydrochloride. Chromatography with chloroform-methanol was carried out only once.

Example XXXII

6-[3-(3-Methylsuccinimid-3-yl)-1-propoxycarbonyl aminosulfonylamino] fluorescein

3-Methyl-3-(3-hydroxy-1-propyl)succinimide (22 mg., 0.13 mmole) was dissolved in 1 ml. of dichloromethane and added dropwise to a stirred, ice-chilled solution of 14 ml. (0.16 mmole) of chlorosulfonyl isocyanate in 1 ml. of dichloromethane. The ice bath was removed and stirring was continued for 20 minutes. The solvent was removed and the residue redissolved in 0.5 ml. acetonitrile plus 0.2 ml. dimethylformamide. 1/2 Of this solution was allowed to react with 20 mg. (0.065 mmole) of 6-aminofluorescein and 0.0052 ml. (0.065 mmole) of pyridine for 7 hours at room temperature. The product was isolated by chromatography on a silica gel thin layer with benzene-ethylacetate-acetone. A second chromatography employing chloroform-methanol removed the small remaining amounts of by-products.

Example XXXIII

6-{4-[3-(3-Methyl-3-succinimidyl)propylamino]-6-chloro-1,3,5-triazin-2-yl amino} fluorescein

3-Methyl-3-(3-aminopropyl)succinimide (3 mg.) and 6-(4,6-dichloro-1,3,5-triazin-2-ylamino) fluorescein (8.5 mg.) were dissolved in 0.2 ml. methanol with the addition of 0.0063 ml. of triethylamine and the mixture was stirred at room temperature over night. The product was isolated by chromatography on silica gel thin layer plates with chloroform-methanol and then with a mixture of benzene, ethyl acetate amd acetone.

Example XXXIV

6-[2-(3-Methyl-3-ethyl-1-succinimidyl)-1-ethoxy carbonylamino sulfonylamino] fluorescein

3-Methyl-3-ethyl-1-(2-hydroxyethyl)succinimide (373 mg., 2mmoles) was dissolved in 5 ml. of dichloromethane and a solution of 0.173 ml. (2 mmoles) of chlorosulfonyl isocyanate in 1 ml. dichloromethane was added. The mixture was stirred for 10 minutes, filtered to remove a small amount of precipitate, and the solvent was removed. The residue was redissolved in 0.70 ml. of pyridine, and 0.10 ml. of this solution was mixed with a solution of 104 mg. (0.3 mmole) of 6-aminofluorescein in 0.10 ml. pyridine. The reaction was allowed to proceed for 4 hours at room temperature, and the product was isolated by preparative thin layer chromatography, developing twice with chloroform-methanol.

Example XXXV

6-[3-(3-Methyl-3-ethyl-1-succinimidyl)-1-propoxycarbonylamino sulfonylamino] fluorescein

3-Methyl-3-ethyl-1-(3-hydroxypropyl)succinimide (20 mg., 0.1 mole) in 1.0 ml. of dichloromethane was added dropwise to a stirred, ice-chilled solution of 18 mg. of chlorosulfonyl isocyanate (0.125 mmoles) in 1 ml. of dichloromethane. After 10 minutes, half of the solutions was aliquoted out and the solvent was removed. The residue was taken up in 150 ml. of acetonitrile and 50 ml. of pyridine, and 17.3 mg. (0.05 mmoles) of 6-aminofluorescein was added. The mixture was stirred at room temperature for 2 1/2 hours and streaked onto a silica gel preparative thin layer chromatography plate. Development with benzene-ethyl acetate-acetone gave a major product which was repurified with chloroform-methanol to yield the tracer.

Example XXXVI

5-[3-(3-Methyl-3-ethyl-1-succinimidyl)-1-propylamino thiocarbonylamino] fluorescein

A solution of 7.5 mg. of 3-methyl-3-ethyl-1-(3-aminopropyl)succinimide hydrochloride and 11.7 mg. fluorescein-5-isothiocyanate in 0.10 ml. of pyridine was stirred at room temperature for 4 hours. The product was isolated by preparative thin layer chromatography, developing with benzene-ethylacetate-acetone.

Example XXXVII

6-[3-(3-Methyl-3-ethyl-1-succinimidyl)propyl carbonyl amino] fluorescein

3-(3-Methyl-3-ethyl-1-succinimidyl)propionic acid (15 mg.) and triethylamine (9.2 ml.) were dissolved in 0.2 ml. dimethylformamide and chilled in an ice bath. Isobutyl chloroformate, (9 mg.) was stirred in. The mixture was allowed to warm gradually to room temperature over 2 hours and 23 mg. of 6-aminofluorescein was added. The coupling was allowed to proceed overnight and the solvent was removed in vacuo. The residue was taken up in methanol and chromatographed on silica gel thin layer plates with 5:1 chloroform-methanol, taking the band just above unreacted fluoresceinamine.

Example XXXVIII

5[-4-{3-(3-Methyl-3-ethyl-1-succinimidy)-1-propylamino}--6-chloro-1,3,5-triazin-2-ylamino] fluorescein

A solution of 10.5 mg. of 3-methyl-3-ethyl-1-(3-aminopropyl) succinimide hydrochloride, 23.8 mg. of 5-(4,6-dichloro-1,3,5-triazin-2-ylamino) fluorescein and 0.006 ml. of triethylamine in 0.50 ml. of methanol was stirred overnight at room temperature. A second 0.006 ml. portion of triethylamine was added and stirring was continued at room temperature for 4 days. The product was isolated by chromatography on silica gel thin layer plates, developing with chloroform-methanol.

Example XXXIX

5-[4-(3,3-Dimethyl-1-succinimidyl)-1-butyl carbonylamino] fluorescein

3,3-Dimethyl-1-(4-carboxy-1-butyl)succinimide (14.5 mg.) and triethylamine (8.3 ml.) were dissolved in 200 ml. of dimethylformamide the mixture was chilled in an ice-salt bath, and 8.7 mg. of isobutyl chloroformate was stirred in. Stirring was continued as the ice melted over a period of 2 hours, and 22 mg. of 5-aminofluorescein was added. The mixture was stirred at room temperature overnight, and the product was isolated by preparative thin layer chromatography, developing with 5 to 1 chloroform-methanol.

Example XL

5-[4-{3-Methyl-3-ethyl-1-succinimidylmethyl} benzenesulfonamido] fluorescein

4-(3-Methyl-3-ethyl-1-succinimidylmethyl)benzene sulfonyl chloride (26 mg.) and 5-aminofluorescein (27.4 mg.) were dissolved in 250 ml. of pyridine and stirred for 45 minutes at room temperature. The product was purified by thin layer chromatography, developing with 4:1 chloroform-methanol and taking the band which migrated just above unreacted aminofluorescein.

Example XLI

5-[4-{2-[2-(3-Methyl-3-ethyl-1-succinimidyl)ethyl] phenylamino}-6-chloro-1,3,5-triazin-2-ylamino] fluorescein

2-[2-(3-Methyl-3-ethyl-1-succinimidyl)ethyl] aniline (28 mg., 0.1 mmole), triethylamine (20 mg., 0.2 mmole) and 5-[4,6-dichloro-1,3,5-triazin-2-yl-amino] fluorescein (42.5 mg., 0.08 mmoles) were taken up in 1 ml. of methanol and allowed to stand at room temperature for 24 hours. The product was purified by thin layer chromatography, eluting with chloroform-methanol.

Example XLII

6-[4-(3-Methyl-3-ethyl-1-succinimidyl)-1-butyl carbonyl amino] fluorescein

A solution of 48 mg. of 3-methyl-3-ethyl-1-(4-carboxybutyl) succinimide and 28 ml. of triethylamine in 0.50 ml. of dry dimethylformamide was chilled in an ice-salt bath, and 26 ml. of isobutyl chloroformate was added. The mixture was stirred as it warmed gradually to room temperature over the course of 2 hours. Half of this solution was added to 35 mg. of 6-aminofluorescein and the solution was stirred at room temperature overnight. The product was purified by chromatography on silica gel thin layer plates with chloroform-methanol as developing solvents. A second chromatography under the same conditions gave the pure tracer.

Example XLIII

5-[(3-Methyl-3-ethyl-1-succinimidyl)pentacarbonyl amino]fluorescein

A solution of 9.2 mg. of 1-(5-carboxypentyl)-3-methyl-3-ethyl succinimide and 0.0048 ml. of triethylamine in 0.20 ml. of dimethyl-formamide was chilled in an ice-water bath and treated with 4.7 ml. of isobutyl chloroformate. The mixture was stirred for 1 hour, 20 minutes as the ice melted. Fluoresceinamine (5-isomer, 12.5 mg.) was added, and stirring was continued at room temperature overnight. The tracer was purified by thin-layer chromatography with 5:1 chloroform-methanol, taking the band which migrated faster than unreacted fluoresceinamine.

Example XLIV

5-[4-{4-(3-Methyl-3-ethyl-1-succinimidyl)phenylamino-6-chloro-1,3,5-triazin-2-yl}amino] fluorescein

4-(3-Methyl-3-ethyl-1-succinimidyl) aniline (2.3 mg.) and 5-[4,6-dichloro-1,3,5-triazin-2-ylamino] fluorescein (5.3 mg.) were dissolved in 0.20 ml. of methanol and stirred at room temperature overnight. The pure product was obtained by thin layer chromatography employing chloroform-methanol as solvent.

Example XLV

6-[4-{3-(3-Methyl-3-ethyl-1-succinimidyl)phenylamino-6-chloro-1,3,5-triazin-1-yl} amino] fluorescein

3-(3-Methyl-3-ethyl-1-succinimidyl aniline (3.7 mg.) and 6-[4,6-dichloro-1,3,5-triazin-2-ylamino] fluorescein (8 mg.) were stirred at room temperature in 0.25 ml. of methanol for 36 hours. The pure product was obtained by preparative chromatography on a silica gel thin layer plate developed with chloroform-methanol, taking the most mobile band.

## Claims

**1.** A compound comprising the structure:

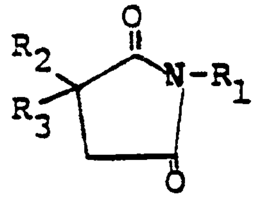

wherein

$R_1$ is -H or -R-Z-Q;

$R_2$ is $-CH_2$-R-Z-Q when $R_1$ is H and $R_2$ is methyl or ethyl when $R_1$ is -R-Z-Q;

$R_3$ is methyl or ethyl;

Q is a poly(aminoacid) or another immunologically active carrier selected from natural proteins, synthetic poly(aminoacids) having a sufficient number of available carboxylate groups, carbohydrates, yeasts and polysacharides, or fluorescein or a fluorescein derivative;

Z is NH, CO, CS, $SO_2$, C=NH, O or a single bond when Q is said fluorescein or fluorescein derivative and NH-NH, NH, N=N or $CH_2$ when Q is said poly(aminoacid) or other immunologically active carrier; and

R is a group consisting of from 0 to 8 carbon atoms arranged in a straight or branched chain or in a phenylene or alkylenephenylene moiety, and with the proviso that when $R_1$ is R-Z-Q, Q is an amino derivative of fluorescein, Z is CO and R is a normal alkylene chain, then R must contain at least three carbon atoms.

**2.** The compound of claim 1 wherein Q is bovine serum albumin.

**3.** The compound of claim 1 wherein Q is a carbonyl, an amino, an amido, an amidino, an urea, a thiourea, a carbamate, a thiocarbamate, a triazinylamino, a sulfonamido or a (carboxyamino)sulfonamido derivative of fluorescein.

**4.** The compound of claim 1 wherein Q is 4-chloro-6-(fluorescein-5-ylamino)-1,3,5-triazin-2-yl,4-chloro-6-(fluorescein-6-ylamino)-1,3,5-triazin-2-yl,4-methoxy-6-(fluorescein-5-ylamino)-1,3,5-triazin-2-yl,4-methoxy-6-(fluorescein-6-ylamino)-1,3,5-triazin-2-yl ,fluorescein-5-ylcarbonyl fluorescein-6-ylcarbonyl, (fluorescein-6-yl)amino, or (fluorescein-5-yl)amino.

**5.** An antibody raised by a compound according to claim 1 wherein Q is a poly(aminoacid) or another immunologically active carrier selected from natural proteins, synthetic poly(aminoacids) having a sufficient number of available carboxylate groups, carbohydrates, yeasts and polysacharides.

**6.** A method for making an immunogen comprising the step of coupling a precursor of the formula

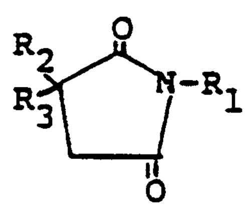

wherein:

$R_1$ is -H or -R-X;

$R_2$ is $-CH_2$-R-X when $R_1$ is H and $R_2$ is methyl or ethyl when $R_1$ is -R-X;

$R_3$ is methyl or ethyl;

X is $NH_2$, Cl, Br, I, OH or CHO; and

R is a group consisting of from 0 to 8 carbon atoms arranged in a straight or branched chain or in a phenylene or an alkylenephenylene moiety

with a poly(aminoacid) or another immunologically active carrier selected from natural proteins,

synthetic poly(aminoacids) having a sufficient number of available carboxylate groups, carbohydrates, yeasts and polysacharides.

7. The method of claim 6 wherein the poly(aminoacid) is bovine serum albumin.

8. The method of claim 6 wherein the reaction is carried out by water-soluble carbodiimide coupling.

9. A method for making a tracer comprising the step of coupling a precursor of the formula:

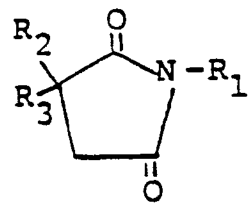

wherein
$R_1$ is -H or -R-Y;
$R_2$ is $-CH_2$-R-Y when $R_1$ is H and $R_2$ is methyl or ethyl when $R_1$ is -R-Y;
$R_3$ is methyl or ethyl;
Y is $-NH_2$, -COOH, $-SO_3H$, $SO_2Cl$, -SH, -CHO, -CN or -OH;
R is a group consisting of from 0 to 8 carbon atoms arranged in a straight or branched chain or in a phenylene or alkylenephenylene moiety, with the proviso that when $R_1$ is -R-Y, R is a normal alkylene chain and Y is COOH, then R must contain at least three carbon atoms,
with fluorescein or a derivative of fluorescein.

10. A process for measuring concentration of ethosuximide which comprises the steps of:
(a) contacting a fluid sample, in the presence of a buffer to achieve and maintain the pH, with an ethosuximide antiserum and with a compound according to claim 1, containing fluorescein in the open tautomeric form, capable of producing a detectable fluorescence polarization response to the presence of the ethosuximide antiserum:
(b) passing plane polarized light through the resulting solution from step (a) to obtain a fluorescence polarization response; and
(c) detecting the fluorescence polarization response of the solution of step (b) as a measure of the amount of ethosuximide in the sample.

**Revendications**

1. Un composé ayant la structure :

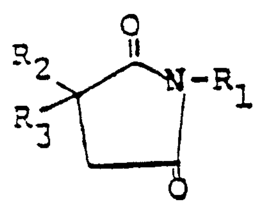

dans laquelle :
$R_1$ et -H ou -R-Z-Q ;
$R_2$ est $-CH_2$-R-Z-Q lorsque $R_1$ est H et $R_2$ est un groupe méthyle ou éthyle lorsque $R_1$ est -R-Z-Q ;
$R_3$ est un groupe méthyle ou éthyle ;
Q est un poly(aminoacide) ou un autre support immunologiquement actif choisi parmi les protéines naturelles, les poly(aminoacides) synthétiques ayant un nombre suffisant de groupes carboxylates

disponibles, les hydrates de carbone, les levures et les polysaccharides, ou la fluorescéine ou un dérivé de la fluorescéine ;

Z est NH, CO, CS, $SO_2$, C = NH, O ou une liaison simple lorsque Q est la fluorescéine ou un dérivé de la fluorescéine, et NH-NH, NH, N = N ou $CH_2$ lorsque Q est ledit poly(aminoacide) ou un autre support immunologiquement actif ; et

R est un groupe constitué de 0 à 8 atomes de carbone agencés en une chaîne linéaire ou ramifiée ou en une moitié phénylène ou alkylènephénylène, et à la condition lorsque $R_1$ est R-Z-Q, Q est un dérivé amino de la fluorescéine, Z est CO et R est une chaîne alkylène normale, alors R doit contenir au moins 3 atomes de carbone.

**2.** Le composé selon la revendication 1 selon lequel Q est de l'albumine du sérum bovin.

**3.** Le composé selon la revendication 1 selon laquel Q est un dérivé carbonyle, un dérivé amino, un dérivé amido, un dérivé amidino, un dérivé urée, un dérivé thiourée, un dérivé carbamate, un dérivé thiocarbamate, un dérivé triazinylamino, un dérivé sulfonamido ou un dérivé (carboxyamino)sulfonamido de la fluorescéine.

**4.** Le composé selon la revendication 1 selon lequel Q est un groupe 4-chloro-6-(fluorescéine-5-ylamino)-1,3,5-triazine-2-yle, 4-chloro-6-(fluorescéine-6-ylamino)-1,3,5-triazine-2-yle-4-méthoxy-6-(fluorescéine-5-ylamino)-1,3,5-triazine-2-yle, 4-méthoxy-6-(fluorescéine-6-ylamino)-1,3,5-triazine-2-yle, fluorescéine-5-ylcarbonyle, fluorescéine-6-ylcarbonyle, (fluorescéine-6-yl)amino, ou (fluorescéine-5-yl)amino.

**5.** Un anticorps dont la formation est provoquée par un composé selon la revendication 1 dans lequel Q est un poly(aminoacide) ou un autre support immunologiquement actif choisi parmi les protéines naturelles, les poly(aminoacides) synthétiques ayant un nombre suffisant de groupes carboxylates disponibles, les hydrates de carbone, les levures et les polysaccharides.

**6.** Une méthode pour préparer un immunogène comprenant l'étape de couplage d'un précurseur de formule

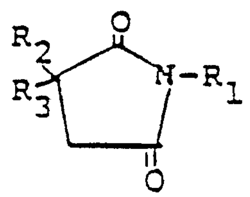

dans laquelle :

$R_1$ est -H ou -R-X ;

$R_2$ est $-CH_2$-R-X lorsque $R_1$ est H et $R_2$ est un groupe méthyle ou éthyle lorsque $R_1$ est -R-X ;

$R_3$ est un groupe méthyle ou éthyle ;

X est $NH_2$, Cl, Br, I, OH ou CHO ; et

R est un groupe constitué de 0 à 8 atomes de carbone agencés en une chaîne linéaire ou ramifiée ou en une moitié phénylène ou alkylènephénylène

avec un poly(aminoacide) ou un autre support immunologiquement actif choisi parmi les protéines naturelles, les poly(aminoacides) synthétiques ayant un nombre suffisant de groupes carboxylates disponibles, les hydrates de carbone, les levures et les polysaccharides.

**7.** La méthode selon la revendication 6, selon laquelle le poly(aminoacide) est l'albumine du sérum bovin.

**8.** La méthode selon la revendication 6 selon laquelle la réaction est conduite par couplage d'un carbodiimide soluble dans l'eau.

**9.** Une méthode de préparation d'un traceur comprenant l'étape de couplage d'un précurseur de formule :

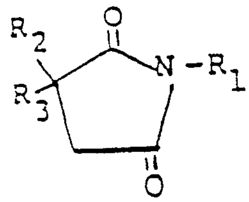

dans laquelle :

$R_1$ est -H ou -R-Y ;

$R_2$ est $-CH_2$-R-Y lorsque $R_1$ est H et $R_2$ est un groupe méthyle ou éthyle lorsque $R_1$ est -R-Y ;

$R_3$ est un groupe méthyle ou éthyle ;

Y est $-NH_2$, -COOH, $-SO_3H$, $SO_2Cl$, -SH, -CHO, -CN ou -OH ;

R est un groupe constitué de 0 à 8 atomes de carbone agencés en une chaîne linéaire ou ramifiée ou en une moitié phénylène ou alkylènephénylène, à la condition que lorsque $R_1$ est -R-Y, R est une chaîne alkylène normale et Y est COOH, alors R doit contenir au moins 3 atomes de carbone,

avec la fluorescéine ou un dérivé de la fluorescéine.

**10.** Un procédé pour mesurer la concentration de l'éthosuximide qui comprend les étapes suivantes :

(a) mise en contact d'un échantillon de fluide en présence d'un tampon pour obtenir et maintenir le pH, avec un antisérum éthosuximide et avec un composé selon la revendication 1, contenant de la fluorescéine sous une forme tautomère ouverte, capable de produire une réponse de polarisation de la fluorescence détectable à la présence de l'antisérum éthosuximide :

(b) passage de la lumière polarisée dans un plan à travers la solution résultante de l'étape (a) pour obtenir une réponse de polarisation de la fluorescence : et

(c) détection de la réponse de polarisation de la fluorescence de la solution de l'étape (b) en tant que mesure de la quantité d'éthosuximide dans l'échantillon.

**Patentansprüche**

**1.** Verbindung der Struktur

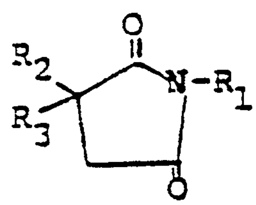

worin

$R_1$ -H oder -R-Z-Q ist;

$R_2$ $-CH_2$-R-Z-Q ist, wenn $R_1$ H ist und $R_2$ Methyl oder Ethyl ist, wenn $R_1$ R-Z-Q ist;

$R_3$ Methyl oder Ethyl ist;

Q eine Poly(aminosäure) oder ein anderer immunologisch wirksamer Träger, ausgewählt aus natürlichen Proteinen, synthetischen Poly(aminosäuren) mit einer ausreichenden Zahl an verfügbaren Carboxylatgruppen, Kohlenhydraten, Hefen und Polysacchariden, oder Fluorescein oder ein Fluoresceinderivat ist;

Z NH, CO, CS, $SO_2$, C=NH, O oder eine Einfachbindung ist, wenn Q Fluorescein oder das Fluoresceinderivat ist, und NH-NH, NH, N=N oder $CH_2$ ist, wenn Q die Poly(aminosäure) oder der andere immunologisch wirksame Träger ist; und

R eine aus 0 bis 8 geradkettig oder verzweigt oder in einer Phenylen- oder Alkylenphenyleneinheit angeordneten Kohlenstoffatomen bestehende Gruppe ist, mit der Maßgabe, daß wenn $R_1$ R-Z-Q ist, Q ein Aminoderivat von Fluorescein ist, Z CO ist und R eine normale Alkylenkette ist, dann R wenigstens drei Kohlenstoffatome enthalten muß.

2. Verbindung nach Anspruch 1, worin Q Rinderserumalbumin ist.

3. Verbindung nach Anspruch 1, worin Q ein Carbonyl-, Amino-, Amido-, Amidino-, Harnstoff-, Thioharnstoff-, Carbamat-, Thiocarbamat-, Triazinylamino-, Sulfonamido- oder (Carboxyamino)-sulfonamidoderivat von Fluorescein ist.

4. Verbindung nach Anspruch 1, worin Q 4-Chlor-6-(fluorescein-5-ylamino)-1,3,5-triazin-2-yl, 4-Chlor-6-(fluorescein-6-ylamino)-1,3,5-triazin-2-yl, 4-Methoxy-6-(fluorescein-5-ylamino)-1,3,5-triazin-2-yl, 4-Methoxy-6-(fluorescein-6-ylamino)-1,3,5-triazin-2-yl, Fluorescein-5-ylcarbonyl, Fluorescein-6-ylcarbonyl, (Fluorescein-6-yl)amino oder (Fluorescein-5-yl)amino ist.

5. Antikörper, erzeugt durch eine Verbindung nach Anspruch 1, worin Q eine Poly(aminosäure) oder ein anderer aus natürlichen Proteinen, synthetischen Poly(aminosäuren) mit einer ausreichenden Zahl an verfügbaren Carboxylatgruppen, Kohlenhydraten, Hefen und Polysacchariden ausgewählter immunologisch wirksamer Träger ist.

6. Verfahren zur Herstellung eines Immunogens, das als Schritt die Kupplung eines Vorläufers der Formel

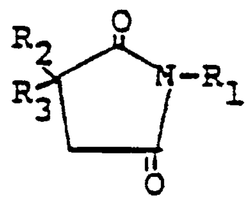

worin

R $_1$ -H oder -R-X ist;
$R_2$ $-CH_2$-R-X ist, wenn $R_1$ H ist, und $R_2$ Methyl oder Ethyl ist, wenn $R_1$ -R-X ist;
$R_3$ Methyl oder Ethyl ist;
X $NH_2$, Cl, Br, I, OH oder CHO ist; und
R eine aus 0 bis 8 geradkettig oder verzweigt oder in einer Phenylen- oder einer Alkylenphenyleneinheit angeordneten Kohlenstoffatomen bestehenden Gruppe ist,

mit einer Poly(aminosäure) oder einem anderen aus natürlichen Proteinen, synthetischen Poly(aminosäuren) mit einer ausreichenden Zahl an verfügbaren Carboxylatgruppen, Kohlenhydraten, Hefen und Polysacchariden ausgewählten immunologisch wirksamen Träger umfaßt.

7. Verfahren nach Anspruch 6, worin die Poly(aminosäure) Rinderserumalbumin ist.

8. Verfahren nach Anspruch 6, worin die Reaktion durch Kupplung mit wasserlöslichem Carbodiimid durchgeführt wird.

9. Verfahren zur Herstellung eines Tracers, welches als Schritt die Kupplung eines Vorläufers der Formel

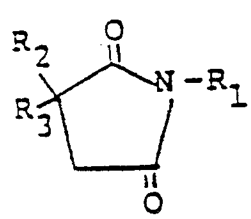

worin

$R_1$ -H oder -R-Y ist;
$R_2$ $-CH_2$-R-Y ist, wenn $R_1$ H ist, und $R_2$ Methyl oder Ethyl ist, wenn $R_1$ -R-Y ist;
$R_3$ Methyl oder Ethyl ist;

Y -$NH_2$, -COOH, -$SO_3H$, -$SO_2Cl$, -SH, -CHO, -CN oder -OH ist;

R eine aus 0 bis 8 geradkettig oder verzweigt oder in einer Phenylen- oder Alkylenphenyleneinheit angeordneten Kohlenstoffatomen bestehende Gruppe ist, mit der Maßgabe, daß wenn $R_1$ -R-Y ist, R eine normale Alkylenkette ist und Y COOH ist, dann R wenigstens drei Kohlenstoffatome enthalten muß, mit Fluorescein oder einem Fluoresceinderivat.

**10.** Verfahren zur Messung der Konzentration von Ethosuximid, welches als Schritte umfaßt:

(a) das Inberührungbringen einer flüssigen Probe in Gegenwart eines Puffers zur Einstellung und Beibehaltung des pH mit einem Ethosuximidantiserum und mit einer Verbindung nach Anspruch 1, die Fluorescein in der offenen tautomeren Form enthält, die eine nachweisbare Fluoreszenzpolarisationsantwort auf die Gegenwart von Ethosuximidantiserum geben kann;

(b) Durchleiten von planar polarisiertem Licht durch die resultierende Lösung aus Schritt (a) zwecks Erhalt einer Fluoreszenzpolarisationsantwort; und

(c) Nachweis der Fluoreszenzpolarisationsantwort der Lösung aus Schritt (b) als Maß für die Menge an Ethosuximid in der Probe.

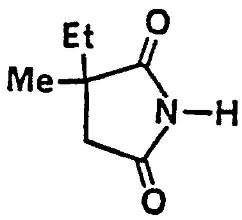

Fig. 1

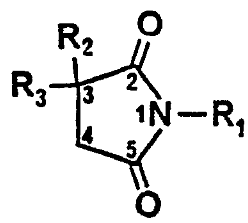

Fig. 2

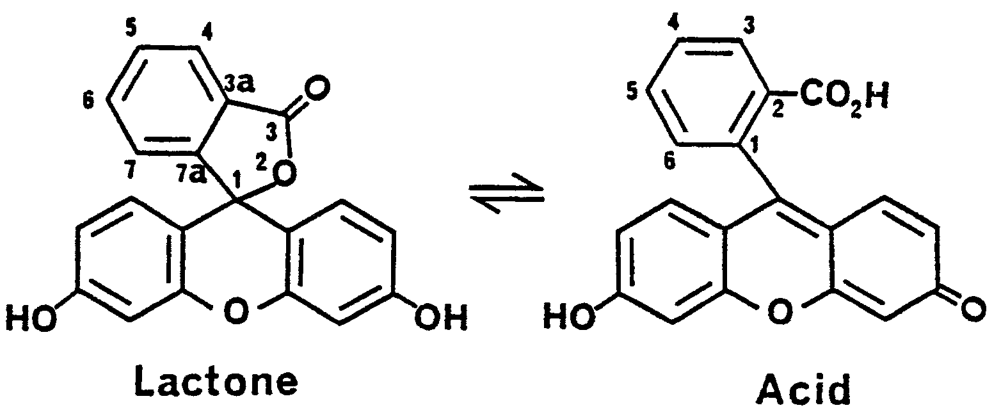


Fig. 3

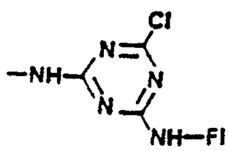

Fig. 4-1

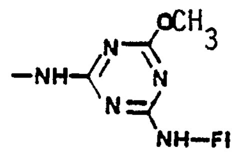

Fig. 4-2

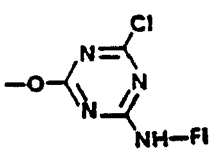

Fig. 4-3

-NH-CO-Fl
Fig. 4-4

-CO-NH-Fl
Fig. 4-5

-C(NH)-NH-Fl
Fig. 4-6

-NH-CO-NH-Fl
Fig. 4-7

-NH-CS-NH-Fl
Fig. 4-8

-O-CO-NH-Fl
Fig. 4-9

-O-CS-NH-Fl
Fig. 4-10

$-SO_2-NH-Fl$
Fig. 4-11

$-O-CO-NH-SO_2-NHFl$
Fig. 4-12

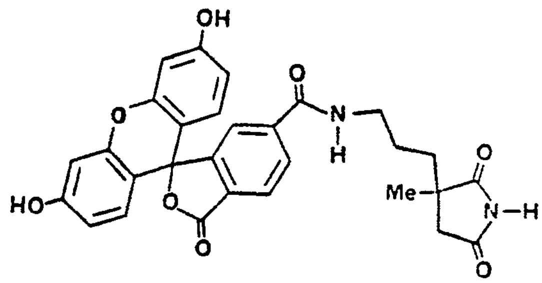

Fig. 5

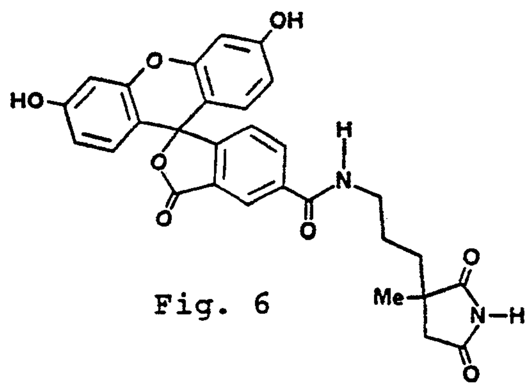

Fig. 6

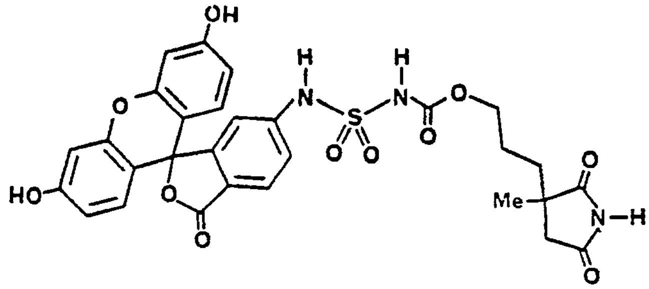

Fig. 7

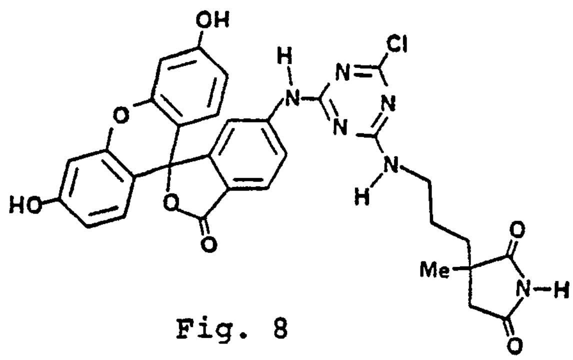

Fig. 8

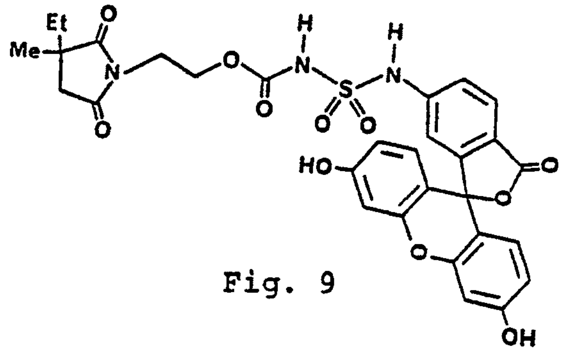

Fig. 9

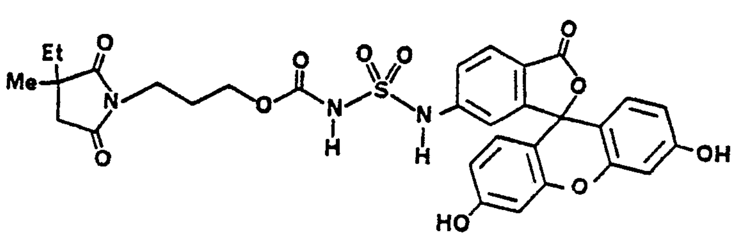

Fig. 10

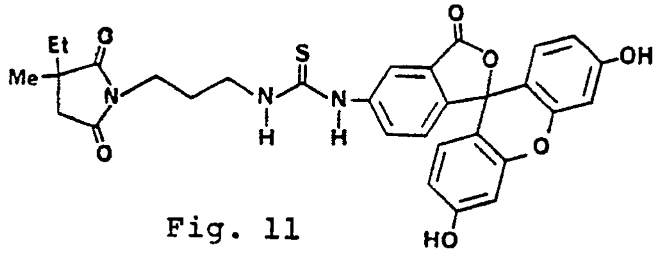

Fig. 11

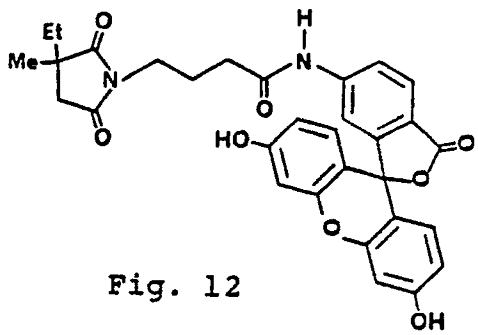

Fig. 12

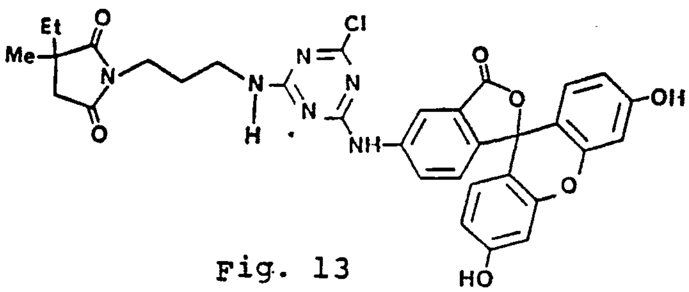

Fig. 13

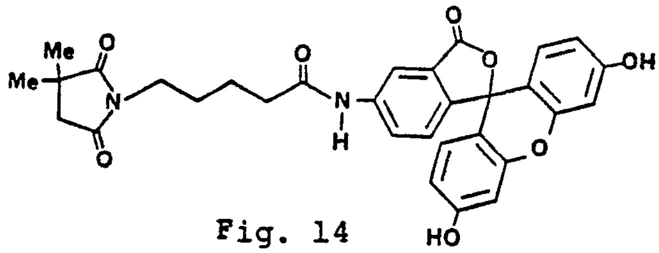

Fig. 14

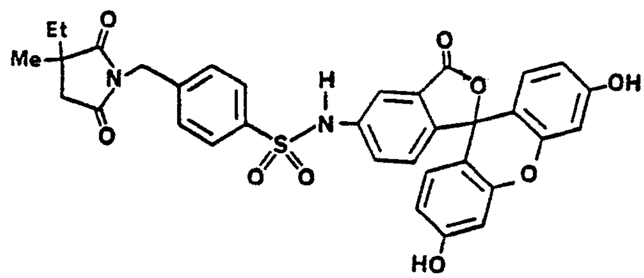

Fig. 15

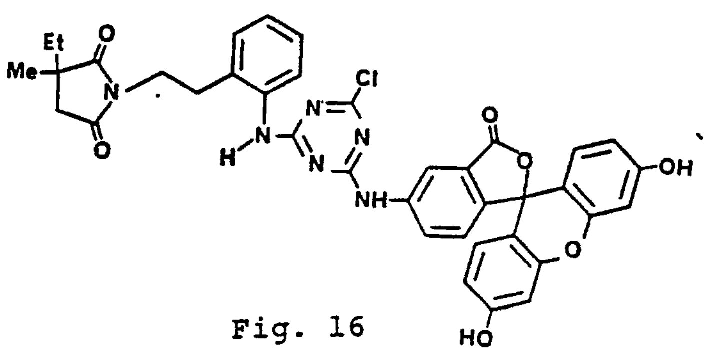

Fig. 16

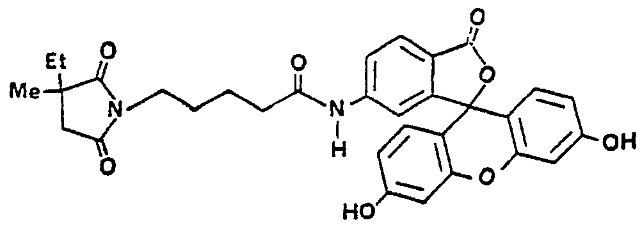

Fig. 17

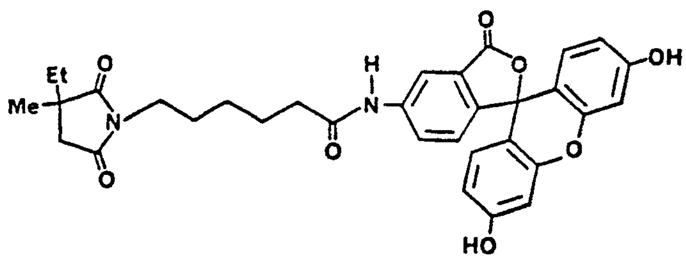

Fig. 18

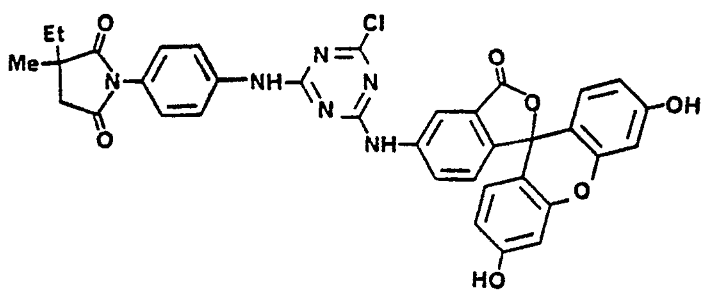

Fig. 19

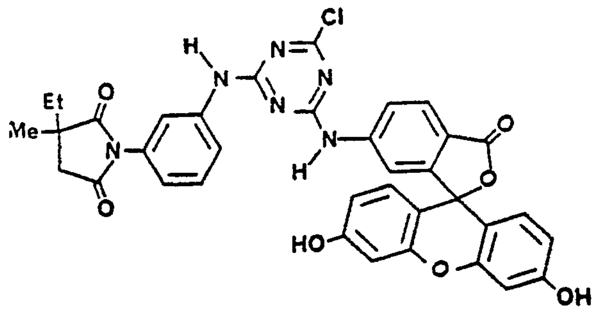

Fig. 20

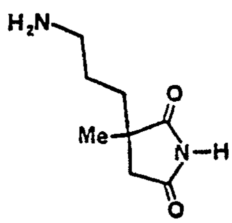

Fig. 21

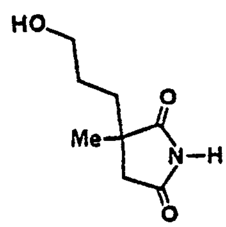

Fig. 22

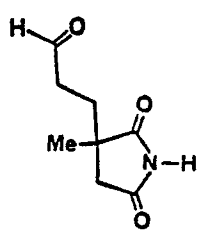

Fig. 23

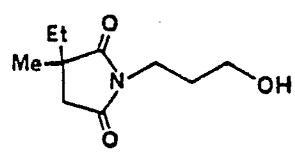

Fig. 24